# EUROPEAN PATENT SPECIFICATION

(11) **EP 3 342 384 B1**
(45) Date of publication and mention of the grant of the patent: **03.03.2021**
(21) Application number: 16850763.0
(22) Date of filing: 21.06.2016
(51) Int. Cl.: A61F 13/49, A61F 13/495

(54) **DISPOSABLE WEARING ARTICLE**
EINWEGKLEIDUNGSARTIKEL
ARTICLE DE PROTECTION JETABLE

(30) Priority: 30.09.2015 JP 2015194923
(43) Date of publication of application: 04.07.2018
(73) Proprietor: Unicharm Corporation, Ehime 799-0111 (JP)
(72) Inventor: INOUE, Takuya, Kanonji-shi Kagawa 769-1602 (JP); TAKINO, Shunsuke, Kanonji-shi Kagawa 769-1602 (JP); MAKI, Hideaki, Kanonji-shi Kagawa 769-1602 (JP)
(74) Representative: Dolleymores
(86) International application number: PCT/JP2016/068322
(87) International publication number: WO 2017/056586

(56) References cited:
- JP-A- H0 970 414
- JP-A- 2013 022 346
- JP-A- 2013 022 346
- JP-A- 2013 118 962

## Description

### [Technical Field]

The present invention relates to a disposable wearing article.

### [Background Art]

Conventionally, Patent Literature 1 discloses a disposable wearing article having, in its worn state, a vertical direction, a lateral direction, a front-back direction orthogonal to these directions, a body facing surface, a non-body facing surface, and including a front waist region, a back waist region and a crotch region extending between the front and back waist regions, and being provided with an absorbent structure for absorbing body fluids.

The wearing article is provided with a belt-like expansion portion on the non-body facing surface of the front waist region. Both lateral side edges of the expansion portion is integrally fixed to both side seal edges of the front waist region and a main portion lying between both the lateral side edges thereof is not fixed to the front waist region.

### [Citation List]

### [Patent Literature]

Unexamined Patent Application Publication 2011-189068(JP 2011-189068 A)

JP 2013 022346 A relates to a pants-type absorbent article having a handle for lifting the article towards the waist of wearer.

### [Summary of Invention]

### [Technical Problem]

In the wearing article disclosed in Patent Literature 1, the main portion lying between both the lateral side edges of the expansion portion is not fixed to the front waist region, and when an upper end of the front waist region is pulled up, a force applied to the upper end of the front waist region leads to result in separating the expansion portion from the front waist region.

An object of the present invention is to improve the conventional article and provide a wearing article capable of moving upward an absorbent structure when an upper end of a back waist region is pulled up.

### [Solution to Problem]

The present invention provides the disposable wearing article of independent claim 1. The dependent claims specify preferred but optional features.

The present invention is directed to a disposable wearing article having a vertical direction, a lateral direction, a body facing surface and a non-body facing surface in a worn state.

The disposable wearing article according to the present invention includes a front waist region, a back waist region, and a crotch region extending between the front and back waist regions and including an absorbent structure for absorbing body fluids; the back waist region includes a back waist belt extending in the lateral direction, and a pocket extending in the lateral direction and facing the back waist belt in a front-back direction at a middle in the lateral direction of the back waist belt and openable downward; the pocket includes a pocket outer wall, a pocket inner wall facing the back waist belt and the pocket outer wall and lying therebetween, the back waist belt and the pocket inner wall are contiguous to each other at a contiguous lower edge of the back waist belt and the pocket inner wall, and the pocket inner wall and the pocket outer wall are contiguous to each other at contiguous upper edge of the pocket inner wall and the pocket outer wall; the back waist region includes both first joining areas spaced apart from each other in the lateral direction and joining the back waist belt and the pocket inner wall to each other; and the first joining areas includes respective inside parts located on respective inner sides of any of respective imaginary lines extending upward along respective lateral side edges of the absorbent structure and respective outside parts located on respective outer sides of the respective imaginary lines and the respective lateral side edges of the absorbent structure respectively in the lateral direction.

The back waist region includes both second joining areas spaced apart from each other in the lateral direction and joining the pocket inner wall and the pocket outer wall to each other; and the second joining areas include respective inside parts located on respective inner sides of any of respective imaginary lines extending upward along respective lateral side edges of the absorbent structure and respective outside parts located on the respective outer sides of the respective imaginary lines and the respective lateral side edges of the absorbent structure respectively in the lateral direction. According to such wearing article, when a force to pull up the back waist region has been applied to the back waist region, the absorbent structure can be easily moved upward, and the pocket can be inhibited from getting out of shape.

Respective outside parts are located on respective lateral side areas in the back waist region, and the first and second joining areas extend continuously from the respective outside parts to the respective inside parts . According to such wearing article, the pocket can be inhibited from getting out of shape.

The pocket inner wall is arranged with at least one elastic member to elastically stretch and contract the pocket inner wall in the lateral direction. According to such wearing article, the stiffness of the pocket inner wall is improved, and a force to pull up the back waist region upward can be transmitted to the absorbent structure.

The pocket outer wall is arranged with at least one elastic member to elastically stretch and contract the pocket outer wall. According to such wearing article, the stiffness of the pocket outer wall can be improved, and a force to pull up the back waist region can be transmitted to the absorbent structure.

The pocket further includes both pocket lateral side walls formed of the pocket inner and outer walls and folded inward in the lateral direction. According to such wearing article, the pocket provided with both the pocket lateral side walls makes it possible to increase an amount of body exudates to be collected and contained.

### [Effects of Invention]

With the wearing article according to one or more embodiments, the respective first joining areas includes the respective inside parts and the respective outside parts, and when a force to pull up the back waist region has been applied to the back waist region, the absorbent structure can be moved upward, and the pocket can be inhibited from getting out of shape.

### [Brief Description of Drawings]

The drawings illustrate specific embodiments of the present invention including optional and preferred embodiments as well as essential features of the invention, in which
Fig. 1 is a back perspective view showing a first embodiment of a disposable diaper as one example of a disposable wearing article according to the present invention in the configuration it would assume when put on a wearer;
Fig. 2 is a partially cutaway flat-opened view of the diaper in which respective elastic members are stretched in vertical and lateral directions;
Fig. 3(a) is a partially cutaway exploded view of a pocket base sheet, Fig. 3(b) is a perspective view showing a folding process of the pocket base sheet, and Fig. 3 (c) is a perspective view showing the pocket base sheet folded along first and second folding lines;
Fig. 4 is a partially cutaway exploded perspective view of a back waist region;
Fig. 5 is an exploded perspective view of part of the diaper;
Fig. 6 is a schematic cross-sectional view along V1-V1 line in Fig. 1;
Fig. 7 is a schematic cross-sectional view along V11-V11 line in Fig. 1;
Fig. 8 is a perspective view showing a state when an upper end at a middle part of a back waist region of the diaper is pulled up;
Fig. 9 is a perspective view showing a second embodiment of the diaper according to the present invention in the configuration it would assume when put on a wearer;
Fig. 10 (a) is a partially cutaway flat-opened perspective view of the pocket base sheet, Fig. 10(b) is a perspective view showing a folding process of the pocket base sheet, Fig. 10 (c) is a perspective view showing the pocket base sheet folded along first and second folding line, and Fig. 10 (d) is a perspective view showing the pocket base sheet folded a third folding line;
Fig. 11 is a cross-sectional plan view of an upper end of the back waist region when the diaper is not worn;
Fig. 12 is a partially enlarged view similar to Fig. 4 of the back waist region showing a modification reference example 1 which does not form part of the present invention;
Fig. 13 is a cross-sectional view similar to Fig. 2 showing a modification embodiment 2;
Fig. 14 is a cross-sectional view similar to Fig. 6 showing a modification embodiment 3; and
Fig. 15 is a transverse cross-sectional view of a back end of an absorbent chassis showing a modification embodiment 4.

### [Description of Embodiments]

### <First Embodiment>

A pull-on (pant-shaped) disposable diaper 10 as one example of a disposable wearing article has a vertical direction Y, a lateral direction X, a thickness (3-dimensional) direction Z, a vertical direction Y in a worn state, a vertical axis P bisecting a dimension in the lateral direction X, and a lateral axis Q bisecting a dimension in the vertical direction Y. As used herein, the term "overlapping with each other in planar view of a diaper 10" refers to overlapping with each other in a thickness direction Z. Also, as used herein, the term "a pull-on disposable diaper" refers to an undergarment which is pulled up to the wearer's waist while the wearer's both legs are being inserted into both horses of a pant-like diaper, and it does not matter whether with or without or lengths of its horses.

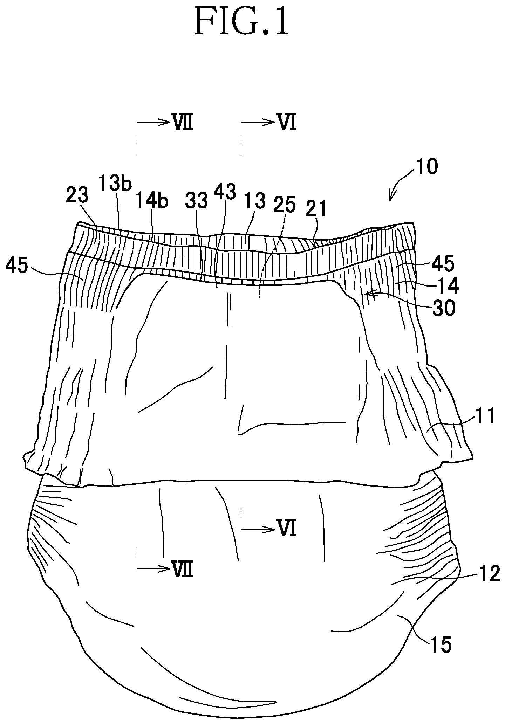

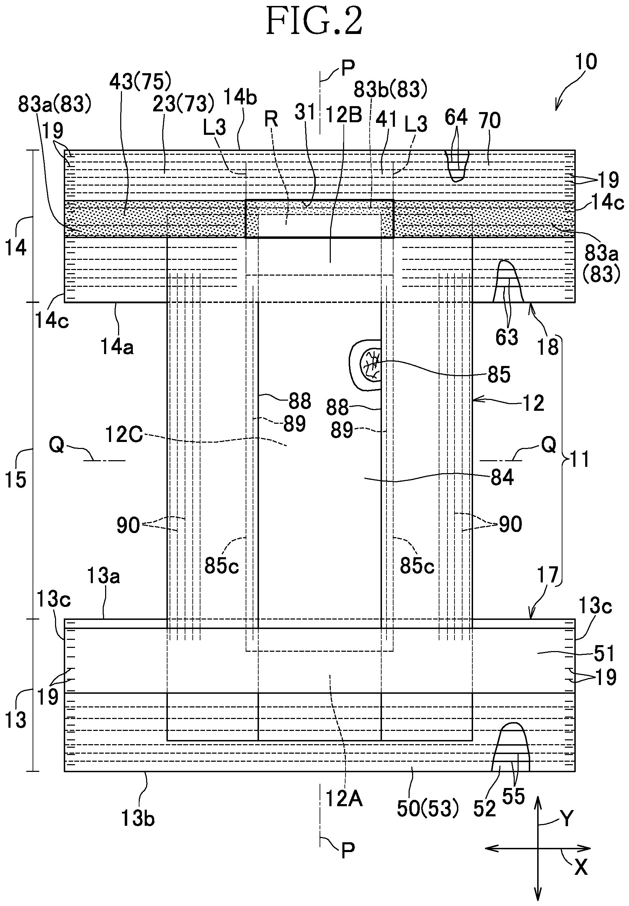

With reference to Figs. 1 and 2, the disposable diaper 10 includes a body facing surface, a non-body facing surface, an elastic waist panel 11 assumable an annular shape extending in a waist circumference direction, an absorbent chassis 12, a front waist region 13, a back waist region 14, and a crotch region 15 extending between the front and back waist regions 13, 14. The diaper 10 is symmetrical about the vertical axis P. The elastic waist panel 11 includes a front waist panel 17 lying in the front waist region 13 and a back waist panel 18 lying in the back waist region 14.

The front and back waist regions 13, 14 have respectively lateral rectangles contoured by lower edges 13a, 14a extending in the lateral direction X, upper edges (front and back waist opening edges) 13b, 14b extending in the lateral direction X, both lateral side edges extending in the vertical direction Y between the respective lower and upper edges 13a, 14a; 13b, 14b. the respective both lateral side edges 13c of the front waist region 13 and the respective both lateral side edges 14c of the back waist region 14 are joined to each other by more than one discrete spaced bonding sites 19 at both lateral side seam, whereby both lateral side parts of the diaper 10 is formed, and a waist opening 21 and a pair of leg openings are defined. The bonding sites may be made by well-known bonding means such as heat embossment/debossment processing or ultrasonic processing.

### <Pocket>

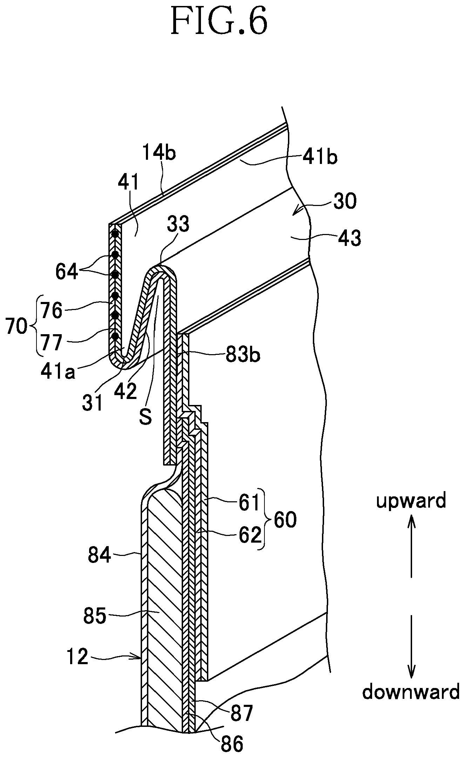

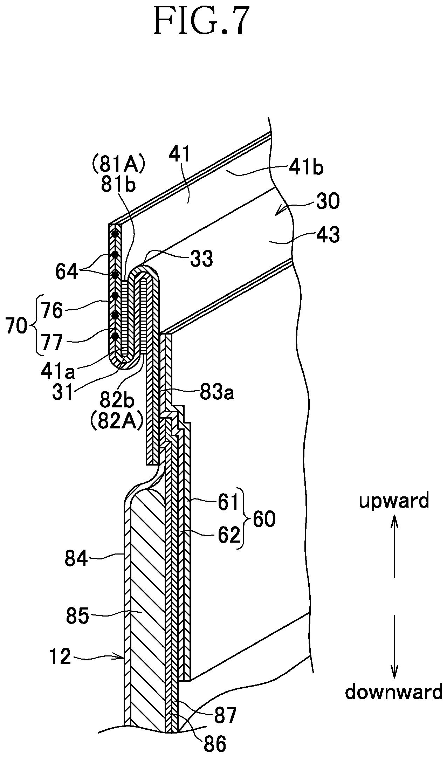

With reference to Figs. 1, 6 and 7, the diaper 10 includes a back waist belt 23 to come in contact with the body of a wearer on the waist opening side of the back waist region 14, and a portion 41 of the back waist belt 23 is located approximately in the middle of the back waist region 14 in the lateral direction X and has an upper edge 41b and a lower edge 41a. The diaper 10 further includes a pocket 30 facing the non-body facing surface of the back waist belt 23. The pocket 30 has a predetermined area extending in the lateral direction X centered on the vertical axis P and is openable downward, i.e., toward the crotch region 15. The back waist belt 23 and the pocket 30 are located on the waist opening side of the back waist region 14. As used herein, the term "on the waist opening side of the back waist region 14" refers to an area lying between the upper edge 14b and an imaginary line approximately bisecting a dimension in the vertical direction Y of the back waist region 14.

The pocket 30 includes a pocket inner wall 42 and a pocket outer wall 43 have a contiguous upper edge 33 contiguous to each other and face each other in a front-back direction of the diaper 10. The pocket inner wall 42 has a contiguous lower edge 31 contiguous to the lower edges 41a of the back waist belt 23 and lies between the pocket outer wall 43 and the back waist belt 23, and the pocket outer wall 43 lies between the contiguous upper edge 33 and part of the back waist region 14 lower than the lower edge 41a of the back waist belt 23 in the vertical direction Y. Both lateral side areas 45 of the pocket inner and outer walls 42, 43 are overlappedly joined to each other, and both joined lateral side areas 45 are fixed to both lateral areas of the back waist belt 23 facing the former. Both lateral side areas 45 each extend at a predetermined distance from both lateral side seams having the bonding sites 19 toward the vertical axis P, and the pocket 30 has a lateral length in the lateral direction X to the extent that the pocket 30 is openable at the middle of the back waist region 14 at least without being openable between both lateral side seams. The contiguous lower and upper edges 31, 33 are respective folds between the back waist belt 23 and the pocket inner wall 42 and between the pocket inner and outer walls 42, 43.

### <Elastic Waist Panel>

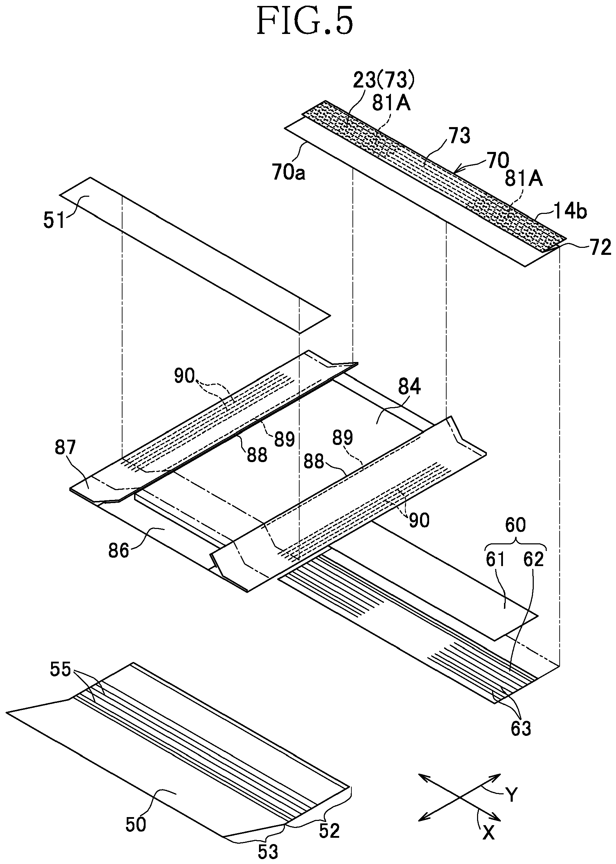

With reference to Figs. 2 and 5, the front waist panel 17 includes a front waist sheet 50. The front waist sheet 50 includes a main section 52 on which a front end 12A of the absorbent chassis 12 is put and a sub-section 53 contiguous to the main section 52. The sub-section 53 is fixedly put on the main section 52 and the front end 12A of the absorbent chassis 12 put on the inner surface of the main section 52 while being folded along a folding line between the main and sub-sections 52, 53. Between the main section 52 and the sub-section 53, a plurality of string- or strand-like front waist elastic members 55 extending in the lateral direction X are secured in a contractible manner under tension.

A front elastic strip 51 contractible in the lateral direction X is disposed on an area of the front waist sheet 50 lying on the side of the crotch regions 16 at which the waist elastic members 55 are not located. The front elastic strip 51 is fixed on part of the front end 12A of the absorbent chassis 12 and the main section 52 and part of the main section 52 on the side of the body facing surface of the front waist panel 17.

The back waist panel 18 includes a waist sheet 60 constructed of an inner sheet 61 lying on the body facing surface and an outer sheet 62 lying on the non-body facing surface, and a plurality of string-or strand-like lower back waist elastic members 63 extending in the lateral direction X are secured in a contractible manner under tension. The back waist region 14 is attached with a pocket base sheet 70 along the upper edge thereof in a folded state as described later.

### <Pocket Base Sheet>

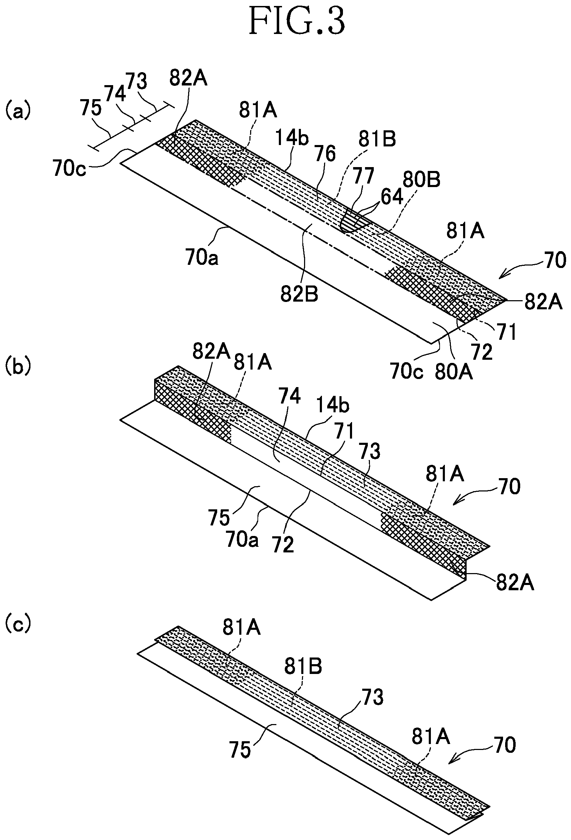

With reference to Fig. 3(a), the pocket base sheet 70 has a lateral rectangle contoured by a lower edge 70a and an upper edge 14b (corresponding to the back waist opening edge) both extending in the lateral direction X and both lateral side edges 70c extending in the vertical direction X between the lower and upper edges 70a, 14b and has a first surface 80A, a second surface 80B, and a first fold line 71 and a second fold line 72 extending in the lateral direction X parallel to each other. The pocket base sheet 70 has an upper edge area 73 defined between the upper edge 14b and the first fold line 71, an intermediate area 74 defined between the first fold line 71 and the second fold line 72 and a lower edge area 75 defined between the second fold line 72 and the lower edge 70a. On the side of the second surface 80B of the upper edge area 73, a pair of first joining areas 81A opposite to each other in the lateral direction X and, between the first joining areas 81A, a first non-joining area 81B extending in the lateral direction X are respectively defined. On the side of the first surface 80A of the intermediate area 74, a pair of second joining areas 82A opposite to each other in the lateral direction X and, between the second joining areas 82A, a second non-joining area 82B extending in the lateral direction is defined. The first joining area 81A is provided for joining the back waist belt 23 and the pocket inner wall 42 to each other, and the second joining area 82A is provided for joining the pocket inner and outer walls 42, 43 to each other. On the first non-joining area 81B, the back waist belt 23 and the pocket inner wall 42 are not joined to each other, and on the second non-joining area 82B, the pocket inner wall 42 and the pocket outer wall 43 are not joined to each other.

The pocket base sheet 70 includes an inner sheet 76 lying on the body facing surface, an outer sheet 77 lying on the non-body facing surface, and back upper waist elastic members 64 of a plurality of string- or strand-like extending in the lateral direction X secured in a contractible manner under tension between the inner and outer sheets 76, 77 on an upper end area 73. With reference to Fig. 3 (b) and (b), the pocket base sheet 70 is folded along a fold line 71 so that the second surface 80B of the upper edge area 73 and the second surface 80B of the intermediate area 74 may be put in contact with each other and folded along the second fold line 72 so that the first surface 80A of the intermediate area 74 and the first surface 80A of the lower edge area 75 may be put in contact with each other. In this way, a state folded in a generally Z-shape as view in cross-section is maintained.

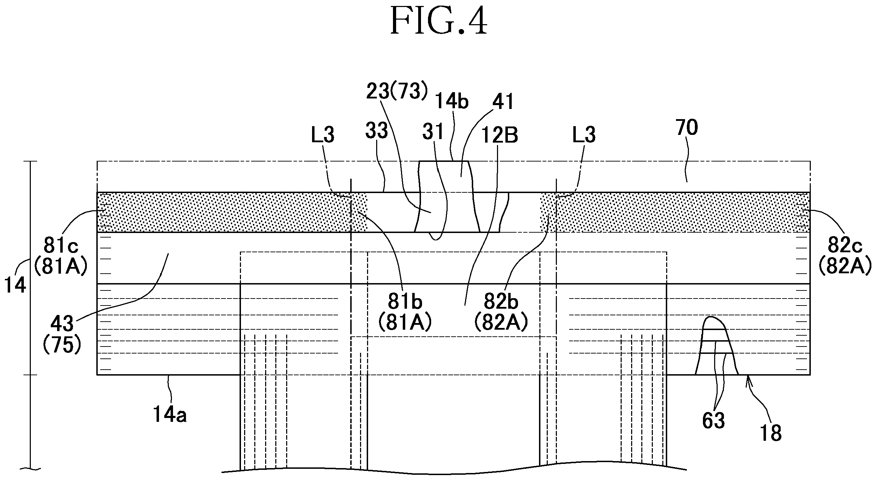

With reference to Fig. 4 (also referring to Fig.3), to show the second joining area 82A, the upper edge area 73 of the pocket base sheet 70 and the intermediate area 74 are cut away on the right side of Fig. 4 for the purpose of illustration, and similarly, to show the first joining area 81A, the upper end part 73 of the pocket base sheet 70 is cut away on the left side of Fig. 4 for the purpose of illustration. The first and second joining areas 81A, 82A each extend continuously in the lateral direction X, have approximately the same dimension in the lateral direction X, and also the first and second non-joining areas 81B, 82B lying between the respective first and second joining areas 81A, 82A have approximately the same dimension in the lateral direction X. The first and second joining areas 81A, 82A each have inside parts 81b, 82b located on the inside of respective imaginary lines L3 extending upward along both lateral side edges of an absorbent structure 85, and outer parts 81c, 82c located on the outside of the respective imaginary lines L3. The outer parts 81c, 82c each overlap with the bonding sites 19 at both side seams of the back waist panel 18. The first and second non-joining areas 81B, 82B are overlapped with each other in planar view. The first and second joining areas 81A, 82A are provided by, for example, well-known joining means such as hot-melt adhesive or heat-sealing.

With reference to Fig. 2, the pocket base sheet 70 is fixed to the absorbent chassis 12 and the back waist panel 18 by the joining area (the third joining area) 83 having a concave shape toward the lateral axis Q. The third joining area 83 has both lateral parts 83a which are opposite to each other in the lateral direction X, has the same shape and size as the first and second joining areas 81A, 82A and is overlapped with the first and second joining areas 81A, 82A in planar view, and a middle part 83b connecting between both lateral parts 83a without being overlapped with the first and second joining areas 81A, 81B. As described above, by the pocket base sheet 70 being folded and fixed, the back waist belt 23 of the back waist region 14 is formed of the upper end part 73, the pocket inner wall 42 is formed of the second non-joining 82B of the intermediate area 74, and the pocket outer wall 43 is formed of part facing the second non-joining area 82B of the lower edge area 75. Further, both lateral sides of the pocket inner wall 42 and both lateral sides of the pocket outer wall 43 are respectively joined together in the respectively overlapped states.

In the present embodiment, the pocket 30 is formed by fixing the pocket base sheet 70 separate of the back waist sheet 60 to the back waist sheet 60; however, as long as the technical effect is achieved, the pocket 30 may be formed by one single sheet having respective parts corresponding to the back waist belt 23 and the back waist sheet 60, or by forming the back waist belt 23 from part of the back waist sheet 60 and joining a separate sheet to the back waist belt 23. Further, the pocket base sheet 70 may be formed of one elastically stretchable/contractible nonwoven fabric sheet or by an elastically stretchable/contractible nonwoven fabric sheet being interposed between the inner and outer sheet 76, 77. Thus, as used herein, the term "the pocket inner wall 42 is contiguous to the contiguous lower edge 41a of the middle part 41 of the back waist belt 23" includes the case where the middle part 41 and the pocket inner wall 42 are formed of the same sheet and folded at the contiguous lower edge 31, and the middle part 41 and the pocket inner wall 42 are formed of respective separate sheets and they are connected to each other. Further, the pocket 30 may be formed by folding an extension formed by lengthening a dimension of the back waist panel in the vertical direction Y.

### <Absorbent Chassis>

With reference to Figs. 2 and 5, the absorbent chassis 12 has a rectangular shape contoured by a front end 12A, a back end 12B, and an intermediate area 12C. The absorbent chassis 12 includes a body side liner 84 made of a liquid permeable fibrous nonwoven fabric, an absorbent structure 85, a leakage barrier sheet 86 made of a liquid impermeable plastic film and disposed on the bottom of the absorbent structure 85, and a cover sheet 87 made of a liquid impermeable or liquid hardly permeable sheet, defining the non-body facing surface of the absorbent chassis 12. The absorbent structure 85 includes a core material made of a mixture of wood fluff pulp and superabsorbent polymer particles, and a wrapping sheet made of liquid permeable and liquid diffusible sheet such as tissue paper, covering the whole of the core material.

The cover sheet 87 has both lateral sides located outside both lateral side edges of the leakage barrier sheet 86. Both the lateral sides are fixed on the body side liner 84 while being folded toward the absorbent structure 85. Both the lateral sides have both ends spaces apart in the vertical direction Y being fixed on the body side liner 84, both proximal edges fixed on both lateral side edges of the body side liner 84, and both distal edges 88 extending in the vertical direction Y in parallel to both the proximal edges . Both the distal edges each have a cuff elastic member 89 of string- or strand-like elastic material extending in the vertical direction Y, secured within a sleeve-like edge formed by the distal edge 88 in a contractible manner under tension. The distal edges 88 each form a barrier cuff by being spaced away from the body side liner 84 toward the body of a wearer so that the distal edges 88 may be put in contact with the wearer's thighs, thereby preventing the leakage of body exudates from the diaper 10. Further, both the lateral sides of the cover sheet 87 are each secured with a plurality of leg elastic members 90 of string- or strand-like elastic materials extending in the vertical direction Y in a contractible manner under tension.

The leg elastic members 90 are composed of a plurality of elastic materials arranged in a state spaced apart by a given dimension in the lateral direction X and define an elastic area having a required width, whereby the elastic area can be put in contact with the wearer's thighs in planar fashion to effectively prevent the side leakage of body exudates. Back end parts of the leg elastic members 90 are overlapped with lower back waist elastic members 63 in the back waist region 14 in planar view. The overlapping of the leg elastic area to act a contractible force of the leg elastic members 90 and the waist elastic area to act a contractible force of the back lower waist elastic members 63 in planar view defines an elastic area to come in contact with the wearer's thighs.

With reference to Figs. 2, 6 and 7, the pocket base sheet 70 is joined to the back waist panel 18 and the back end 12B of the absorbent chassis 12 through the joining area 83 without being joined to the back end 12B of the absorbent chassis 12 between the distal edges 88 of the cover sheet 87. Such joining arrangement defines a back waist space R to collect and contain body exudates having flowed to the back waist region 14 between the non-joining area of the pocket base sheet 70 and the back end 12B of the absorbent chassis 12.

Generally, a relatively few months old baby excretes runny or watery fecal exudates in various situations, during breast-feeding or in a posture of being lifted up in a mother's arms, thus, runny or watery fecal exudates may leak from the back wait opening edge and/or runny or watery fecal exudates may be pressed against and soil the wearer's back. In addition, a space to collect body exudates may be formed between the diaper and the wearer's body when the waist opening of the diaper put on the wearer's body slips down in the front waist region. However, the buttocks have an external shape protruding backward and such an undesired gap will be hardly formed. To ensure that body exudates are collected and temporarily contained, as the diaper 10 according to the present embodiment, a pocket space S having a three-dimensional configuration in the diaper 10 put on the wearer's body should be intentionally configured by the pocket inner wall 42 and the pocket outer wall 43 with the back waist belt 23.

It should be noted here that, while there have been conventionally disclosed diapers provided with a space to collect and contain body exudates having flowed to the back waist region the front, back and/or crotch region, the conventional diapers have not taught diapers such as being configured by the pocket inner wall and the pocket outer wall with the back waist belt in the present invention as far as the present inventors know.

According to the present embodiment, the back waist belt 23 and pocket 30 are located along the rear waist opening part in the back waist region 14 and the back waist belt 23 is put in close contact with the wearer's boy under tensile stress of the upper waist elastic members 64. Thus, body exudates flowing from the crotch region 15 toward the back waist opening is prevented from leaking out and the body exudates of which further flow was inhibited into the pocket space S convexly extending upward and is collected therein to be temporarily contained therein. The inhibited body exudates are guided into the pocket 30 defined on the back waist region 14, whereby prevented are a discomfort feeling and various skin troubles due to the body exudates which may otherwise come in contact with the wearer's body. The pocket 30 is not influenced by stretch/contraction of the back waist belt 23, and thus, the pocket opening should not be closed even when the back waist region 14 is stretched. Further, the pocket 30 is located above the edge (i.e., back edge) of the absorbent structure 85 and the pocket can collect the body exudates which have not been absorbed by the absorbent structure and flows toward the back waist opening part.

The pocket base sheet 70 to form the pocket 30 is partially joined to the back waist region 14 through the joining area 83 extending inward in the lateral direction X from the bonding sites 19 in the side seam and the pocket base sheet 70 itself is located outboard of the leg elastic members 90 and the cuff elastic members in the vertical direction Y without overlapping with them in planar view of the diaper 10. For this reason, even if the contractible force of the leg elastic members 90 and the cuff elastic members 89 indirectly act on the pocket outer wall 43 so as to pull them downward, the pocket outer wall may not be pulled downward, whereby the contiguous upper edge 33 may not collapse and the pocket space S may not get blocked.

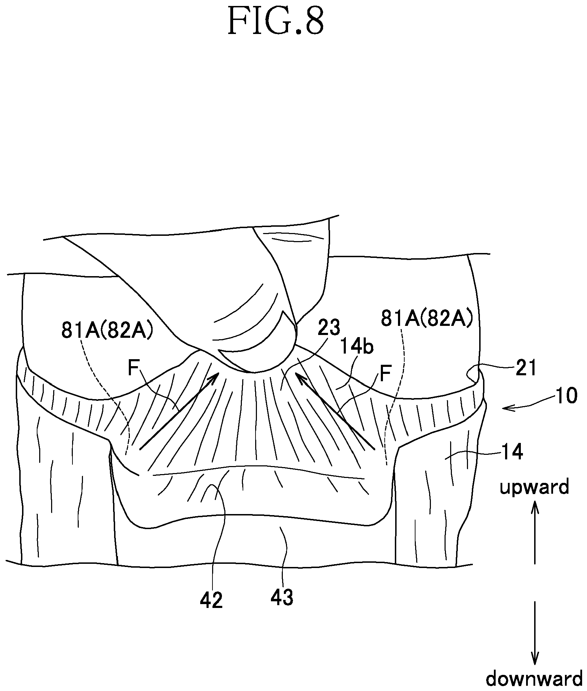

With reference to Figs. 2, 3 and 4, the diaper 10 of the present embodiment includes the first joining area 81A to join the back waist belt 23 and the pocket inner wall 42, and the second joining area 82A to join the pocket inner wall 42 and the pocket outer wall 43. The first and second joining areas 81A, 82A have respectively, in the lateral direction X, inside parts 81b, 82b located on an inside of each imaginary line L3 extending upward along both side edges 85c, and outside parts 81c, 82c located on the outer side of each imaginary line L3. Thus, as shown in Fig. 8, even when a force likely to pull upward is applied to the upper edge 14b of the middle part in the lateral direction X in the back waist region 14, the force is transmitted to the back waist belt 23 and the pocket inner and outer walls 42, 43 together in a direction shown in arrows F. This makes it possible to move the absorbent structure 85 fully upward. Thus, when putting the diaper 10 on the wearer, the whole diaper 10 including the absorbent structure 85 can be pulled fully upward. Further, the first and second joining areas 81A, 82A make it possible to integrate the back waist belt 23 and the pocket inner and outer walls 42, 43, to prevent the back waist belt 23 and the pocket inner wall 42 from separating from each other, and to prevent the pocket inner and outer walls 42, 43 from separating from each other. This enables the pocket 30 to maintain its shape. For such effects, according to the present embodiment, unlike Patent Literature 1 previously stated as an example of prior art, a force applied to the upper edge 14b in the back waist region 14 directly acts on the absorbent structure 85 without acting so as for the back waist belt 23 and the pocket inner wall 42 to separate from each other and so as for the pocket inner wall 42 and the pocket outer wall 42 to separate from each other. This makes it easy to move the absorbent structure 85 fully upward. Although not shown, when a force likely to pull up the upper end 14b on the upper side of the first and second joining areas in the back waist region 14 has been applied to the upper end 14b, the force is transmitted to the absorbent structure 85 through the first and second joining areas 81A, 82A. This makes it possible to exhibit the effect similar to that described above. The diaper 10 is provided with the second joining area 82A because the second joining area 82A makes it possible to integrate the pocket inner and outer walls 42, 43, and to easily pull up the diaper 10 fully upward and to prevent the pocket 30 from getting out of shape.

According to the diaper 10, the first and second joining areas 81A, 82A are approximately equal to each other in their dimensions in the lateral direction X, and also the first and second non-joining areas 81B, 82B located between the first and second joining areas 81A, 82A are approximately equal to each other, and the pocket 30 is located approximately at the middle in the lateral direction X of the back waist region 14. This is preferable in maintaining shapes of the pocket space S and the pocket 30.

According to the diaper 10, the first and second joining areas 81A, 82A extend continuously from the respective inside parts 81b, 82b to the respective outside parts 81c, 82c, and the first and second joining areas 81A, 82A can ensure the inhibition of the pocket 30 from losing shape such that areas of the first and second joining areas 81A, 82A may be as large as possible. In addition, an interval in the lateral direction X between wrinkled lines (vertical lines) formed in the back waist region 14 by the back upper waist elastic members 64 is about 5 to 9 mm. Considering the wrinkled lines, the above description "the first and second joining areas 81A, 82A extend continuously in the lateral direction X from the inside parts 81b, 82b to the outside parts 81c, 82c" includes the case where the non-joining area of a dimension in the lateral direction X of 5 mm or less (a non-joining area in which the back waist belt 23 and the pocket inner wall 42 are not joined to each other, and a non-joining area in which the pocket inner wall 42 and the pocket wall 43 are not joined to each other) is present between the respective inside parts 81b, 82b and the respective outside parts 81c, 82c.

In the present embodiment, the leakage barrier sheet 86 extends from the upper end (rear end) of the absorbent structure 85 to the contiguous upper edge 33 and has the dimension in the lateral direction X larger than the dimension in the lateral direction of the opening of the pocket 30. Such leakage barrier sheet 86 of impermeability is wider than the opening of the pocket and located on the opening of the pocket, thereby preventing the body exudates entering into the pocket space S from exudating out of the bottom (the pocket outer wall) of the pocket 30.

### <Second Embodiment>

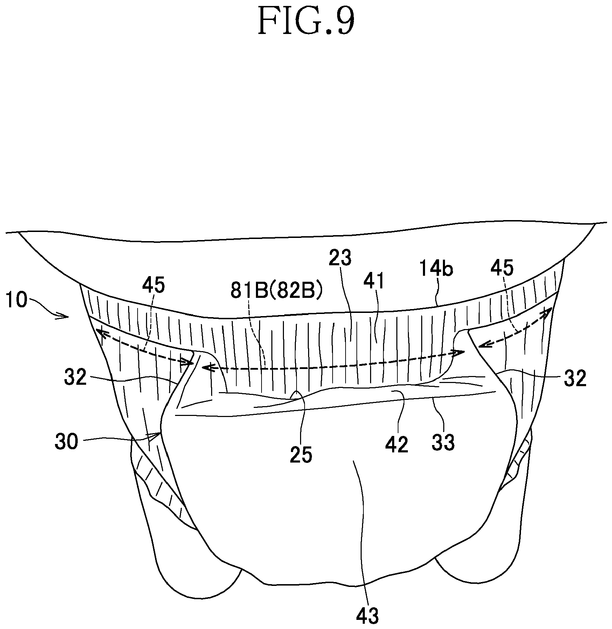

Fig. 9 shows the diaper 10 according to a second embodiment. The pocket 30 of the diaper 10 includes the pocket inner and outer walls 42, 43 and their both lateral sides whose part has both pocket side areas 32 folded inward in a tuck shape in the lateral direction X. The both lateral sides of the pocket inner and outer walls 42, 43 respectively form the pocket lateral side areas 32 by being overlapped with each other, both lateral sides 45 of the pocket inner and outer walls 42, 43 except the both pocket lateral side areas 32 are respectively joined to both lateral sides of the back waist belt 23.

Both lateral side areas 32 are folded inward in the lateral direction X (toward the vertical axis P). Referring to Fig. 9, when the common lower edge 31 and the pocket lateral side areas 32 are opened in a worn state of the diaper 10, a recess 25 opened upward is formed. Both pocket lateral side areas 32 may be formed with more than one wrinkle line extending in the vertical direction Y, including more than one fold formed unintentionally, as long as both pocket lateral side areas 32 are folded in an openable manner.

The pocket 30 has the common lower edge 31 folded downward, both pocket lateral side areas 32 folded inward in the lateral direction X, and the contiguous upper edge 33 folded toward the waist opening 21(upward), they are folded in directions different from one another (three directions), whereby when the back waist belt 23 has been elastically stretched in a waist circumference direction, the both pocket lateral side areas 32 rise or open, and the pocket inner wall 42 moves backward so as to separate from the middle part 41 of the back waist belt 23.

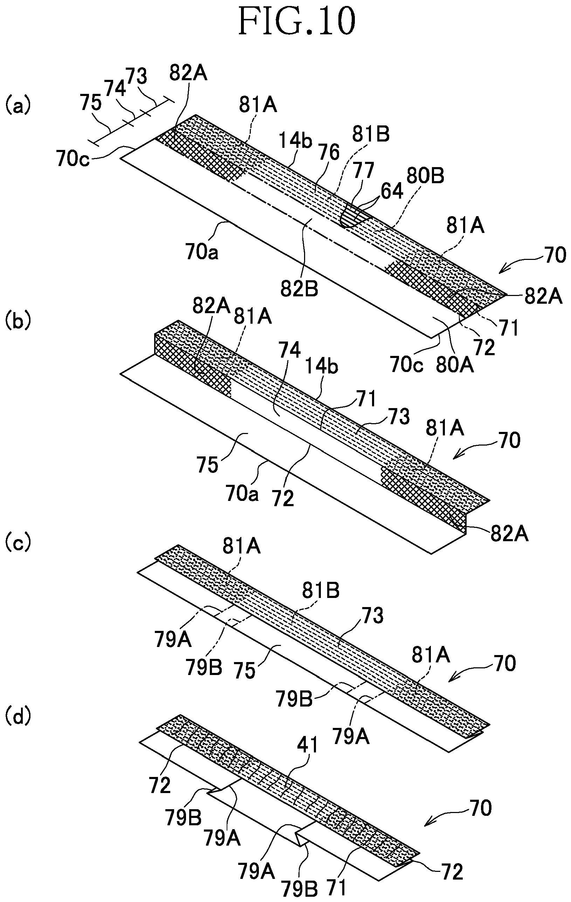

The pocket base sheet 70 is shown in Fig. 10. Figs. 10 (a) and (b) are the same as Figs. 3 (a) and (b), and the description on the former is omitted.

In a state shown in Figs. 10(a)-(c), a force in mutually opposite directions in the lateral direction X is applied to the pocket base sheet 70, and in a state shown in Fig. 10 (d), the upper edge area 73 is contracted by the back upper waist elastic members 64, joined to the back of the intermediate area 74 by the first joining areas 81A after folded on its back along the first fold line 71, such joined upper edge area 73 and intermediate area 74 is joined to the front of the lower edge area 75 by the second joining areas 82A after folded on its front along the second fold line 72 with both lateral sides of the lower edge area 75 folded on the middle of the lower edge area 75 along each pair of third and fourth fold lines 79A, 79B extending in the vertical direction Y along both lateral side edges of the non-joining area 82B, and the intermediate area 74 and the lower edge area 75 except the upper edge area 73 has a cross-sectional Q-shape. A dimension in the lateral direction X of such folded pocket base sheet 70 is approximately equal to that of the back waist region 14 in its natural state (contracted state of the back waist elastic members 64).

With reference again to Fig. 9, in a worn state, both lateral side walls 32 rise or open due to the contraction of the back waist belt 23 in the lateral direction X under a contractible force of the back upper waist elastic members 64, and spaced away from the middle part 41 of the back waist belt 23, whereby the pocket space larger than that of the diaper 10 according to the first embodiment is formed.

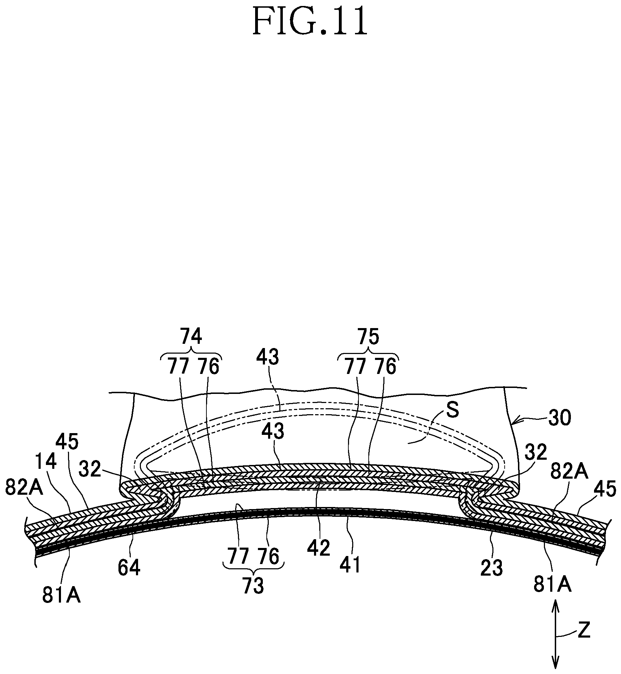

As previously described, the diaper 10 according to the present embodiment includes the pocket inner and outer walls 42, 43 and both pocket lateral side walls 32, as shown in Fig. 11, the pocket 30 exhibits a three-dimensional configuration even in non-worn state and can form a three-dimensional configuration capable of collecting and contain body exudates temporarily in a worn state as expected.

Further, even when a body pressure has been applied to the pocket 30 in a worn state, the middle part 41 of the back waist belt 23 and the pocket inner wall 42 are put in contact with each other with both lateral side walls 32 folded inward, and thus deformation of the pocket base sheet 70 due to body pressure by a wearer and partial change of a thickness of the back waist region 14 thereby may not create a feeling of discomfort against the wearer. Both lateral side walls 32 is capable of folding and stretching (opening) in a direction toward and away from the wearer's body, i.e., in a thickness direction Z, and thus, when the pocket 30 has been released from the body pressure, both lateral side walls 32 rise or open in a direction to separate from the wearer's body to form a three-dimensional pocket space S again.

The diaper 10 has the back upper waist elastic members 64 in the back waist region 14, whereby even when a force likely to press the pocket 30 against the wearer's body has been applied to the pocket 30 repeatedly in a supine position of the wearer or a state held on the back of his or her mother, the pocket 30 is capable of maintaining a three-dimensional configuration while safely repeating. Thus, even when a few months baby such as frequently changes his or her posture or repeat frequent runny or watery fecal exudates is put the diaper 10 on his or her body, it is ensured that the pocket space S collects and contains the runny or watery fecal exudates therein. Further, according to the present embodiment, as previously described, the pocket inner wall 42 has the required dimension in the vertical direction Y, and can function as a top wall of the pocket 30 by moving obliquely rearward to the middle part 41 of the back waist belt 23 due to rising or opening of both lateral side walls 32 from its state folded inward. Thus, the pocket 30 is capable of collecting and containing a larger amount of body exudates compared to conventional diapers not including the top wall and both lateral side walls.

In a worn state, when the back waist belt 23 has been stretched in the lateral direction X (waist circumference direction) to the extent that gathers formed under a contractible force of the back upper waist elastic members 64 disappears, the pocket space S may get blocked by moving of the pocket inner wall 42 toward the middle part 41 of the back waist belt 23 while the folding of both lateral side walls 32 is being released. For this reason, even when the back waist belt 23 has been stretched in the lateral direction X, in order for the pocket 30 to maintain a three-dimension shape, a contraction degree in the lateral direction X of the back waist belt 23 including the middle part 41 (length dimension in the lateral direction X of contracted part) is preferable to be larger than a contraction degree in the lateral direction X of the pocket inner wall 42 and/or the pocket outer wall 43. In such arrangement, for example, even when the back waist belt 23 has been stretched by predetermined magnification compared to a natural state (when contracted) of the back waist belt 23, the pocket inner wall 42 is capable of maintaining a three-dimensional configuration without being put in contact with the pocket outer wall 43 such as the pocket space S gets blocked.

In this regard, preferably, the back waist belt 23 is arranged with elastic members stretchable/contractible in the lateral direction X, while the pocket inner wall 42 and/or the pocket outer wall 43 are arranged with no elastic member or such that they are substantially inelastic. Further, although not shown, when the pocket inner wall 42 and/or the pocket outer wall 43 are arranged with an elastic member stretchable/contractible in the lateral direction X, an elastic member of a contraction degree lower than that of the elastic members 64 arranged on the middle part 41 is preferable to be thereon. In the case where the pocket inner wall 42 is arranged with an elastic member stretchable/contractible in the lateral direction X, a risen or opened state of both lateral side wall 32 and a spaced-apart state of the pocket inner wall 42 from the middle part 41 are maintained against a contractible force of the elastic member when a force likely to rise or open both lateral side walls 32 due to stretching of the middle part 41 acts both lateral side wall 32, the pocket 30 is capable of maintain a three-dimension configuration more safely. Further, in the case where the pocket inner and outer walls 42, 43 are elastically stretchable/contractible, to from the pocket 30 safely, a contraction degree in the lateral direction X of the pocket inner wall 42 is preferable to be higher than that of the pocket outer wall 43.

With reference again to Fig. 9, the upper edge 14b is located above the contiguous upper edge 33 of the pocket 30. Thus, the back waist belt 23 maintains a state put in close contact with the wearer in a worn state, and the leakage of body exudates from the waist opening 21 can be prevented. Further, the middle part 41 and the pocket 30 are formed of the pocket base sheet 70, and dimensions in the lateral direction X of sheet members forming them are equal to each other. Thus, even when the pocket inner wall 42 is arranged with no elastic member, a dimension in the lateral direction X of the pocket inner wall 42 contiguous to the lower edge 41a of the middle part 41 is contracted by contacting under the influence of the back upper waist elastic members 64, whereby the pocket inner wall 42 is formed with multiple gathers (ridges) extending in the vertical direction Y. This makes it possible to improve the stiffness of in the lateral and vertical directions X, Y of the pocket inner wall 42 by functioning such as ribs thereof. Thus, in a state risen or opened of both lateral side walls 32, the pocket 30 can maintain a box-like three-dimensional configuration in a state risen or opened of both lateral side walls 32. Although not shown, the front waist region 13 may be provided with a pocket openable downward similarly to the pocket 30 in the back waist region 14 to collect and contain urine temporarily therein.

### <Modification Reference Example 1>

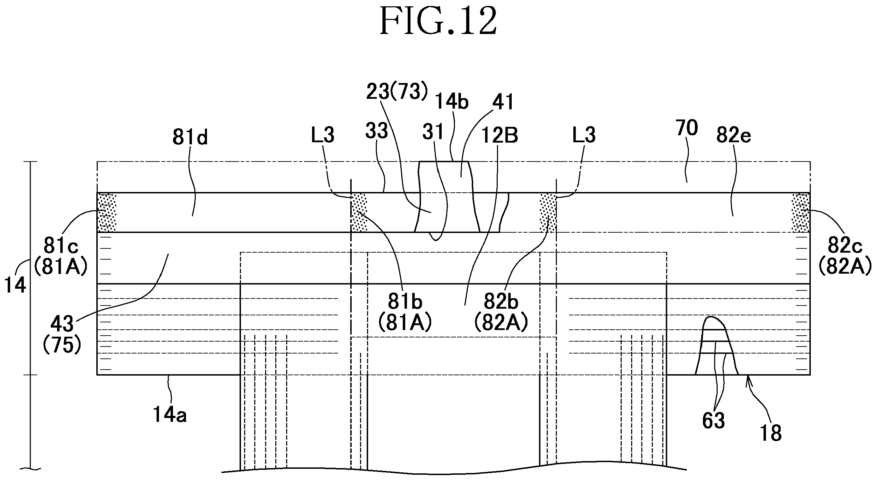

Fig. 12 shows a modification reference example 1 of the diaper 10 according to the first and second embodiments. For convenience of description, for part of the right side in Fig. 12, the upper area 73 and the intermediate area 74 of the pocket base sheet 70 are cutaway to show the second joining area 82A, and for part of the left side in Fig. 12, the upper area 73 of the pocket base sheet 70 is cutaway to show the first joining area 81A. In the present modification reference example, the first joining area 81A and the second joining area 82A have the same shape and the same size, and the first and second joining areas 81A, 82A are disposed so as to overlap with each other in planar view of the diaper 10.

Further, the first and second joining areas 81A, 82A are respectively only formed by inside parts 81b, 82b located on the inner side of an imaginary line L3 extending upward along both lateral side edges 85c of the absorbent structure 85 in the lateral direction X, similarly, outside parts 81c, 82c located on the outer side of the imaginary line L3, and between the inside parts 81b, 82b and the outer side parts 81c, 82c in the lateral direction are respectively formed a non-joining area 81d in which the back waist belt 23 and the pocket inner wall 42 are not joined to each other, and a non-joining area 82e in which the pocket inner wall 42 and the pocket outer wall 43 are not joined to each other. Such diaper 10 makes it possible to reduce areas of the joining areas 81A, 82A, 83 (see Fig. 2) and to decrease an amount of adhesive.

### <Modification Embodiment 2>

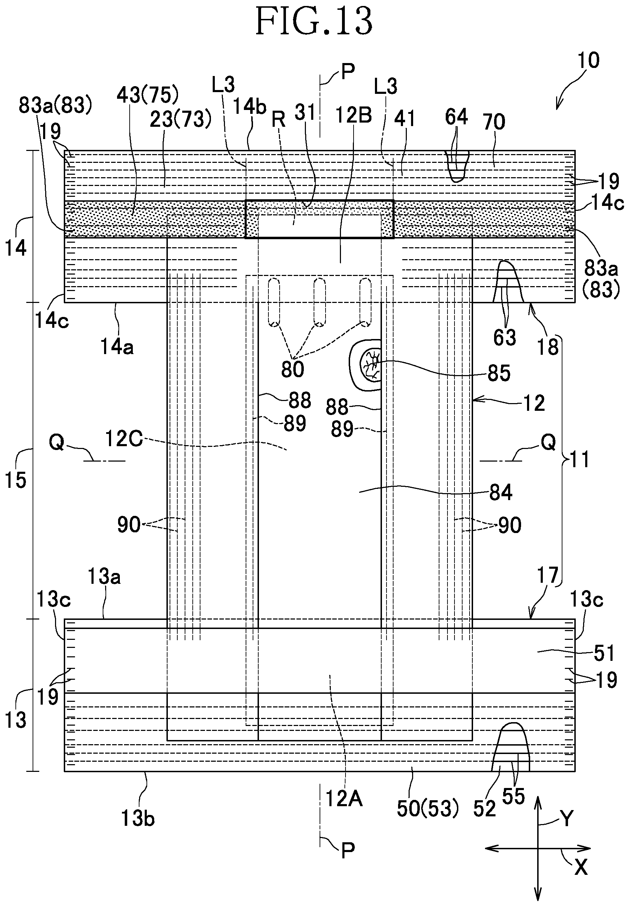

Fig. 13 shows a modification embodiment 2 of the diaper 10 according to the first and second embodiments. Referring to Fig. 13, the absorbent structure 85 of the diaper 10 includes more than one inducing groove 80 extending from the crotch region 15 toward the back waist region 14. The groove 80 may be formed by removing part of absorbent core material of the absorbent structure 85 or by decreasing a mass of the absorbent core material at part corresponding to the inducing groove 80, which may be bottomless groove or bottomed groove. The groove according to the present modification embodiment, the inducing groove 80 has a flat-bottom thinner than other part of the absorbent core material. While the number of the inducing groove 80 is three (3), it may be one (1), and two (2), or four (4) or more. According to such diaper 10 makes it possible to easily deform the back end of the absorbent structure 85 in which the pocket space S is present. This enables the pocket space S to collect and contain runny or watery fecal exudates to further enlarge.

### <Modification Embodiment 3>

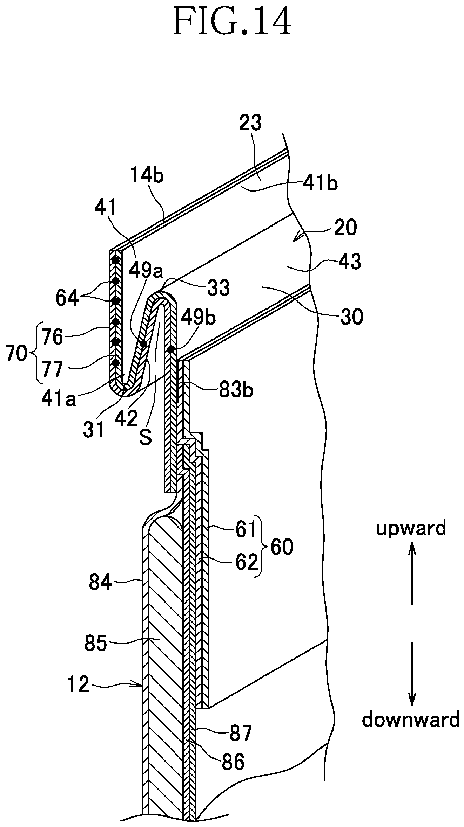

Fig. 14 shows a modification embodiment 3 of the diaper 10 according to the first and second embodiments. Referring to Fig. 14, the pocket base sheet 70 further includes string- or strand-like first and second pocket elastic members 49a, 49b extending in the lateral direction X in a contractible manner under tension between the inner and outer sheets 76, 77.

According to the diaper 10, by an arrangement of first and second pocket elastic members 49a, 49b stretchable in the lateral direction X, a risen or opened state of the pocket lateral side walls 32 and a spaced-apart state of the pocket inner wall 42 from the back waist belt 23 are maintained against a contractible force of the elastic members 49a, 49b when a force likely to rise or open both lateral side walls 32 due to stretching of the middle part 41 acts both lateral side wall 32, and the pocket 30 is capable of maintaining a three-dimension shape more safely. Further, the elastic members 49a, 49b make it possible to increase a mass of the pocket inner and outer walls 42, 43, thereby improving the stiffness of the pocket inner and outer walls 42, 43, and it is ensured that a force to pull up the back waist region 14 upward is capable of transmitted to the absorbent structure. It should be noted that the elastic members 49a, 49b are not necessarily provided on the pocket base sheet 70.

### <Modification Embodiment 4>

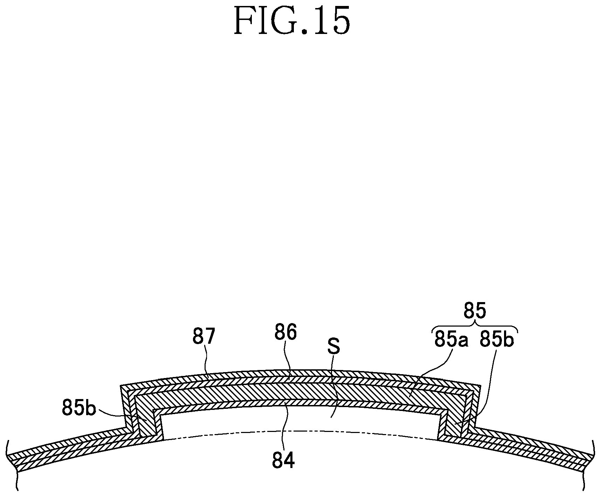

Fig. 15 shows a modification embodiment 4 of the diaper 10 according to the second embodiment. Referring to Fig. 15, by an arrangement of both lateral side walls 32 in the pocket base sheet 70, the absorbent structure 85 at the back end of the absorbent chassis 12 is formed with a main part 85a located at the middle in the lateral direction X and both lateral side wall part 85b. According to
the diaper 10, body exudates such as runny or watery fecal exudates can be absorbed and contained by not only the main part 85 located on the body facing surface of the pocket space S, but also by both lateral side parts 85b located on both lateral sides of the pocket space S.

In the diaper 10 shown in the modification reference example 1 and the modification embodiments 2 to 4 of the first and second embodiments, while there is an interval between the contiguous upper edge 33 and the absorbent structure 85, the absorbent structure 85 may extend to the inside of the pocket space S. when the diaper 10 is provided with such absorbent structure 85, the inside parts 81b, 82b are located on the inner side than both lateral side edges 85c of the absorbent structure 85 and the outside parts 81c,82c are located on the outer side than both lateral side edges 85c of the absorbent structure 85. Further, while an example in which the diaper 10 includes the back waist sheet 60 and the pocket base sheet 70 has been illustrated, the back waist sheet 60 and the pocket base sheet 70 may be a single sheet in which parts corresponding to them is continuous in the singular form. Further, in the diaper 10, an example in which the first joining area 81A and the second joining area 82A are overlapped with each other has been illustrated, it is not necessary to have such an arrangement.

The components of the disposable wearing article 10, which is one example of the article, are not limited to those described in the specification of the present application but other various types of widely used in the relevant technical field may be used. Further, as the basic structure of the article, one example in which the front and back waist regions are constructed of the front and back waist panel 17, 18 separately formed; however, the front and back regions 13, 14 and the crotch region 15 may be formed of a single panel, and the article may be open-type that the front and back waist regions are connected by tape fasteners in use without a pull-on type. The terms "first", "second" and "third" used in the specification of the present application are used merely to distinguish similar members, similar positions or other similar means.

### [Reference signs list]

- 10: disposable wearing article
- 13: front waist region
- 14: back waist region
- 14b: upper edge
- 15: crotch region
- 23: back waist belt
- 30: pocket
- 31: contiguous upper edge
- 32: pocket lateral side wall
- 41: middle part of back waist belt
- 41a: lower edge of middle part
- 42: pocket inner wall
- 43: pocket outer wall
- 45: pocket lateral side wall
- 49a: first pocket elastic member
- 49b: second pocket elastic member
- 81A: first joining area
- 81b: inside part
- 81c: outside part
- 82A: second joining area
- 82b: inside part
- 82c: outside part
- 85: absorbent structure
- 85c: both lateral side edges of absorbent structure
- L3: imaginary line
- X: lateral direction
- Y: vertical direction

## Claims

1. A disposable wearing article (10) having a vertical direction (Y), a lateral direction (X), a body facing surface and a non-body facing surface in a worn state, and comprising a front waist region (13), a back waist region (14) and a crotch region (15) extending between the front and back waist regions and including an absorbent structure (85) for absorbing body fluids, wherein:
the back waist region (14) includes a back waist belt (23) extending in the lateral direction (X) and a pocket (30) adapted for collecting body exudates, the pocket extending in the lateral direction and facing the back waist belt (23) in a front-back direction at a middle in the lateral direction and openable downward;
the pocket (30) includes a pocket outer wall (43), a pocket inner wall (42) facing the back waist belt (23) and the pocket outer wall and lying therebetween, the back waist belt and the pocket inner wall (42) are contiguous to each other at a contiguous lower edge (31) of the back waist belt (23) and the pocket inner wall (42), and the pocket inner wall and the pocket outer wall are contiguous to each other at a contiguous upper edge (33) of the pocket inner wall (42) and the pocket outer wall (43);
the back waist region (14) includes both first joining areas (81A) spaced apart from each other in the lateral direction (X) and joining the back waist belt (23) and the pocket inner wall (42) to each other;
the first joining areas (81A) include respective inside parts (81b) located on respective inner sides of any of respective imaginary lines (L3) extending upward along respective lateral side edges (85c) of the absorbent structure (85) and respective outside parts (81c) located on the respective outer sides of the respective imaginary lines (L3) and the respective lateral side edges (85c) of the absorbent structure in the lateral direction (X);
the back waist region (14) further includes both second joining areas (82A) spaced apart from each other in the lateral direction and joining the pocket inner wall (42) and the pocket outer wall (43) to each other;
the second joining areas (82A) include respective inside parts (82b) located on respective inner sides of any of respective imaginary lines (L3) extending upward along respective lateral side edges (85c) of the absorbent structure (85) and respective outside parts (82c) located on the respective outer sides of the respective imaginary lines and the respective lateral side edges (85c) of the absorbent structure in the lateral direction;
the outside parts (81c,82c) are located on both lateral side areas in the back waist region (14); and
the first and second joining areas (81A,82A) extend continuously from the respective outside parts (81c,82c) to the respective inside parts (81b,82b).

2. The disposable wearing article according to claim 1 wherein the pocket inner wall (42) is arranged with at least one elastic member (49a) to elastically stretch and contract the pocket inner wall in the lateral direction (X).

3. The disposable wearing article according to claim 1 or claim 2 wherein the pocket outer wall (43) is arranged with at least one elastic member (49b) to elastically stretch and contract the pocket outer wall.

4. The disposable wearing article according to any one of claims 1 through 3 wherein the pocket (30) further includes both pocket lateral side walls (32) formed of the pocket inner and outer walls (42,43) and folded inward in the lateral direction(X).

## Patentansprüche

1. Tragbarer Einwegartikel (10), der Folgendes aufweist: eine vertikale Richtung (Y), eine laterale Richtung (X), eine dem Körper zugewandte Oberfläche und eine nicht dem Körper zugewandte Oberfläche in einem getragenen Zustand, und Folgendes umfasst: einen vorderen Taillenbereich (13), einen hinteren Taillenbereich (14) und einen Schrittbereich (15), der sich zwischen dem vorderen und hinteren Taillenbereich erstreckt und eine absorbierende Struktur (85) für das Absorbieren von Körperflüssigkeiten einschließt, wobei:
der hintere Taillenbereich (14) einen hinteren Taillengürtel (23), der sich in der lateralen Richtung (X) erstreckt, und eine Tasche (30), die zum Sammeln von Körperausscheidungen geeignet ist, einschließt, wobei sich die Tasche in der lateralen Richtung erstreckt und dem hinteren Taillengürtel (23) in einer Vorn-hinten-Richtung an einer Mitte in der lateralen Richtung zugewandt ist und nach unten öffenbar ist;
die Tasche (30) Folgendes einschießt: eine Taschenaußenwand (43), eine Tascheninnenwand (42), die dem hinteren Taillengürtel (23) und der Taschenaußenwand zugewandt ist und dazwischen liegt, wobei der hintere Taillengürtel und die Tascheninnenwand (42) an einem fortlaufenden unteren Rand (31) des hinteren Taillengürtels (23) und der Tascheninnenwand (42) aneinander angrenzen und die Tascheninnenwand und die Taschenaußenwand an einem fortlaufenden oberen Rand (33) der Tascheninnenwand (42) und der Taschenaußenwand (43) aneinander angrenzen;
der hintere Taillenbereich (14) beide ersten Verbindungsflächen (81A) einschließt, die voneinander in der lateralen Richtung (X) beabstandet sind und den hinteren Taillengürtel (23) und die Tascheninnenwand (42) miteinander verbinden;
die ersten Verbindungsflächen (81A) Folgendes einschließen: jeweilige Innenseitenteile (81b), die sich auf jeweiligen Innenseiten von jedweder der jeweiligen gedachten Linien (L3) befinden, die sich nach oben entlang jeweiliger lateraler Seitenränder (85c) der absorbierenden Struktur (85) erstrecken, und jeweilige Außenseitenteile (81c), die sich auf den jeweiligen Außenseiten der jeweiligen gedachten Linien (L3) und den jeweiligen lateralen Seitenrändern (85c) der absorbierenden Struktur in der lateralen Richtung (X) befinden;
der hintere Taillenbereich (14) weiter beide zweiten Verbindungsflächen (82A) einschließt, die voneinander in der lateralen Richtung beabstandet sind und die Tascheninnenwand (42) und die Taschenaußenwand (43) miteinander verbinden;
die zweiten Verbindungsflächen (82A) Folgendes einschließen: jeweilige Innenseitenteile (82b), die sich auf jeweiligen Innenseiten von jedweder der jeweiligen gedachten Linien (L3) befinden, die sich nach oben entlang jeweiliger lateraler Seitenränder (85c) der absorbierenden Struktur (85) erstrecken, und jeweilige Außenseitenteile (82c), die sich auf den jeweiligen Außenseiten der jeweiligen gedachten Linien und den jeweiligen lateralen Seitenrändern (85c) der absorbierenden Struktur in der lateralen Richtung befinden;
sich die Außenseitenteile (81c, 82c) auf beiden lateralen Seitenflächen in dem hinteren Taillenbereich (14) befinden; und
sich die ersten und zweiten Verbindungsflächen (81A, 82A) kontinuierlich von den jeweiligen Außenseitenteilen (81c, 82c) zu den jeweiligen Innenseitenteilen (81b, 82b) erstrecken.

2. Tragbarer Einwegartikel nach Anspruch 1, wobei die Tascheninnenwand (42) mit mindestens einem elastischen Element (49a) angeordnet ist, um die Tascheninnenwand in der lateralen Richtung (X) elastisch zu dehnen und zusammenzuziehen.

3. Tragbarer Einwegartikel nach Anspruch 1 oder Anspruch 2, wobei die Taschenaußenwand (43) mit mindestens einem elastischen Element (49b) angeordnet ist, um die Taschenaußenwand elastisch zu dehnen und zusammenzuziehen.

4. Tragbarer Einwegartikel nach einem der Ansprüche 1 bis 3, wobei die Tasche (30) weiter beide lateralen Taschenseitenwände (32) einschließt, die aus der Tascheninnen- und -außenwand (42, 43) gebildet werden und in der lateralen Richtung (X) nach innen gefaltet werden.

## Revendications

1. Article à porter jetable (10) ayant une direction allant dans le sens vertical (Y), une direction allant dans le sens latéral (X), une surface orientée vers le corps et une surface non orientée vers le corps dans un état porté, et comportant une région avant au niveau de la taille (13), une région arrière au niveau de la taille (14) et une région au niveau de l'entrejambe (15) s'étendant entre les régions avant et arrière au niveau de la taille et comprenant une structure absorbante (85) servant à absorber des liquides organiques, dans lequel :
la région arrière au niveau de la taille (14) comprend une ceinture arrière au niveau de la taille (23) s'étendant dans la direction allant dans le sens latéral (X) et une poche (30) adaptée à des fins de collecte d'exsudats organiques, la poche s'étendant dans la direction allant dans le sens latéral et étant orientée vers la ceinture arrière au niveau de la taille (23) dans une direction avant-arrière au niveau d'une partie médiane dans la direction allant dans le sens latéral et ouvrable vers le bas ;
la poche (30) comprend une paroi extérieure de poche (43), une paroi intérieure de poche (42) orientée vers la ceinture arrière au niveau de la taille (23) et vers la paroi extérieure de poche et reposant entre elles, la ceinture arrière au niveau de la taille et la paroi intérieure de poche (42) sont contiguës l'une par rapport à l'autre au niveau d'un bord inférieur contigu (31) de la ceinture arrière au niveau de la taille (23) et de la paroi intérieure de poche (42), et la paroi intérieure de poche et la paroi extérieure de poche sont contiguës l'une par rapport à l'autre au niveau d'un bord supérieur contigu (33) de la paroi intérieure de poche (42) et de la paroi extérieure de poche (43) ;
la région arrière au niveau de la taille (14) comprend deux premières zones d'assemblage (81A) espacées l'une de l'autre dans la direction allant dans le sens latéral (X) et assemblant la ceinture arrière au niveau de la taille (23) et la paroi intérieure de poche (42) l'une par rapport à l'autre ;
les premières zones d'assemblage (81A) comprennent des parties intérieures respectives (81b) situées sur des côtés intérieurs respectifs de l'une quelconque de lignes imaginaires respectives (L3) s'étendant vers le haut le long de bords de côtés latéraux respectifs (85c) de la structure absorbante (85) et des parties extérieures respectives (81c) situées sur les côtés extérieurs respectifs des lignes imaginaires respectives (L3) et les bords de côtés latéraux respectifs (85c) de la structure absorbante dans la direction allant dans le sens latéral (X) ;
la région arrière au niveau de la taille (14) comprend par ailleurs deux deuxièmes zones d'assemblage (82A) espacées l'une de l'autre dans la direction allant dans le sens latéral et assemblant la paroi intérieure de poche (42) et la paroi extérieure de poche (43) l'une par rapport à l'autre ;
les deuxièmes zones d'assemblage (82A) comprennent des parties intérieures respectives (82b) situées sur des côtés intérieurs respectifs de l'une quelconque de lignes imaginaires respectives (L3) s'étendant vers le haut le long de bords de côtés latéraux respectifs (85c) de la structure absorbante (85) et des parties extérieures respectives (82c) situées sur les côtés extérieurs respectifs des lignes imaginaires respectives et les bords de côtés latéraux respectifs (85c) de la structure absorbante dans la direction allant dans le sens latéral;
les parties extérieures (81c, 82c) sont situées sur les deux bords de côtés latéraux dans la région arrière au niveau de la taille (14) ; et
les première et deuxième zones d'assemblage (81A, 82A) s'étendent de manière continue depuis les parties extérieures respectives (81c, 82c) jusqu'aux parties intérieures respectives (81b, 82b).

2. Article à porter jetable selon la revendication 1, dans lequel la paroi intérieure de poche (42) est agencée avec au moins un élément élastique (49a) pour étirer et contracter de manière élastique la paroi intérieure de poche dans la direction allant dans le sens latéral (X).

3. Article à porter jetable selon la revendication 1 ou la revendication 2, dans lequel la paroi extérieure de poche (43) est agencée avec au moins un élément élastique (49b) pour étirer et contracter de manière élastique la paroi extérieure de poche.

4. Article à porter jetable selon l'une quelconque des revendications 1 à 3, dans lequel la poche (30) comprend par ailleurs deux parois de côtés latéraux de poche (32) formées à partir des parois intérieure et extérieure de poche (42, 43) et pliées vers l'intérieur dans la direction allant dans le sens latéral (X).
